# EUROPEAN PATENT APPLICATION

(11) **EP 0 535 629 A1**
(43) Date of publication of application: **07.04.1993**
(21) Application number: 92116724.3
(22) Date of filing: 30.09.1992
(51) Int. Cl.: A61B 5/00

(54) **Integrated medical events emergency rescue system**

(30) Priority: 01.10.1991 IL 99620
(71) Applicant: SHACHAL MEDICAL SERVICES LTD., Tel Aviv 67891 (IL); TADIRAN LTD., Holon 58101 (IL)
(72) Inventor: Kalman, Israel, Kfar Saba 44233 (IL); Elroy, Yoram, Ramat Aviv 69124 (IL); Raviv, Hanan, Tel Aviv (IL)
(74) Representative: Kraus, Walter, Dr.

(57) **Abstract**

A monitoring system for emergency type medical events, such as cardiac or asthma attacks and the like, comprises a medical control center (MCC) where patient data is stored, and a portable rescue monitor unit (CCRM) which enables the patient to transmit to the control center (MCC) data as to his identity and his symptoms and other critical parameters. The system comprises means (19,20,21) for transmitting vocal information from and to the patient and means for initiating the provision of the required aid to the patient.

## Description

### FIELD OF THE INVENTION :

A comprehensive medical rescue system, for use in emergency type medical events is provided. This provides for patient identification , of the patient in need of aid,to a central control center where his identification and medical data are stored, and means for transmitting data to this center, relating to at least one critical medical parameter, indicative of the medical state of the patient. The system enables vocal communication between the patient and the control center, thus enabling the patient to inform the center about his problems, and which enables the center to provide instructions to the patient. The invention is applicable to various types and kinds of medical emergency events, yet it is illustrated mainly with reference to cardiac emergencies, and this is by way of illustration only.

A comprehensive cardiac rescue monitor system (CCRM) is provided which provides information to a central control, termed Monitor and Control Center, (MCC), and which makes possible to provide rapid and effective assistance to a patient in need thereof in the case of signs of an impending or actual cardiac attack. The integrated system provides for the rapid transmission of the required information to central control (MCC), making possible an immediate diagnosis of the nature of the cardiac symptoms, enabling central control to take the necessary steps to provide the required assistance to the patient.

The invention also relates to other types of medical emergencies, such as attacks of asthma or the like, where a patient requires urgent diagnosis of his status and needs, and corresponding immediate assistance. The invention is illustrated with reference to cardiac events, but is not restricted to these, which are given in an illustrative manner.

It is one of the main features of the present invention to provide a system which enables a patient located at any location within certain premises (such as his home) to contact via suitable means, without the manual dialling of a phone, a central control center (MCC);
which system provides means for vocal contact between the patient via home control unit (HCU), and for the transmission via the portable rescue monitor unit (CCRM ), of required physical data of the patient. According to a preferred embodiment of the invention there is provided an emergency button combined with a microphone integrated with it, or located in close proximity to such button, thus providing for clear voice transmission and reception. Furthermore, it is a preferred embodiment of the invention to provide a unique individual code for each patient, thus making sure that such patient is immediately identified at central control (MCC).

The invention is described by way of example, mainly with respect to a cardiac rescue system.

### BACKGROUND OF THE INVENTION:

There exist services which are intended to provide rapid assistence to patients having a cardiac event. Most of these are based on a central control (MCC) unit where the medical record of patient are stored, on the transmission of data such as an ECG from the location of the patient (generally his home) to the control unit, and specially equipped mobile rescue units (ambulances), and preferably Mobile Intensive Care Units, which have the equipment necessary for providing the required immediate assistance, and for transporting the patient to the suitable hospital department where the patient is treated according to his condition.

The novel integrated system provides an improved service to patients in case of a cardiac event, and immediate rescue steps can be initiated in a speed and effective manner.

### SUMMARY OF THE INVENTION:

An integrated medical events emergency rescue system, comprising in combination :
a medical control center (MCC) and means to establish contact by a patient from his premises with such MCC;
a portable rescue monitor unit (CCRM) comprising means for transmitting to MCC unique identifying data about the patient according to his personal code; means for measuring at least one critical parameter indicative of changes of a change in the state of health of the said patient and also for vocal transmission to MCC;
a home control unit (HCU) connected via suitable communication means with said CCRM, said HCU being adapted to receive data from the CCRM and for transmitting same via a phone link to MCC, there existing a link from MCC to HCU via CCRM enabling MCC to provide instructions via vocal means to the patient according to the results of an evaluation carried out at MCC on the basis of the transmitted information and means for initiating any required emergency steps to provide aid to the patient in need thereof.

The integrated comprehensive cardiac rescue monitor (CCRM) fulills the requirement of conveying speedily and without delay the most important information on the real-time status of the patient, including data on his general physical condition as well as his ECG, means for evaluating these data by comparison with the stored medical record at the control center (MCC) and means for providing the required immediate assistance until an intensive care unit reaches the patient, during his transfer to hospital, and transfer to a suitable hospital unit (intensive care unit) or other suitable department.

The system according to the invention comprises means for alerting the control (MCC), informing it that the patient believes that a cardiac event has taken place; means for acquiring certain data (physical data, ECG); means for the immediate transmission of such data to control (MCC); means for evaluating the data, means for initiating emergency treatment and for dispatching a cardiac intensive care unit to pick up and convey the patient to a suitable hospital department.

The system comprises means for establishing reliable and accurate communication with the control unit (MCC); means for the immediate reliable evaluation of the information received, and means for providing a rescue service, depending on the initial diagnosis based on the supplied data.

According to a preferred embodiment of the invention, the rescue system (CCRM) comprises an emergency push button, which is actuated by the patient when he believes that a cardiac event has taken place, or is taking place. Actuation of this button activates the system, and initiates a radio link between the patient and the control center (MCC), including the transmission to the center (MCC) of the personal code of the patient. In practice the radio link is established between the personal unit of the patient and a home control unit (HCU), located at the dwelling of the patient (CCRM), which HCU is connected with a regular phone, so that when the HCU receives an emergency signal, it automatically phones the control center (MCC), establishing an open reliable communications link with the control center, which provides instructions and information to the patient, which receives data on parameters measured in order to provide a diagnosis of the status of the patient, and which permits conveying immediate emergency instructions to the patient as to steps to be taken until a mobile intensive care unit arrives at his dwelling. The system comprises individual patient identification codes, which provides the possibility for a certain HCU to provide links with, and to provide services to more than one CCRMs located in a certain vicinity, within radio transmission range of the HCU and CCRM units.

When the HCU receives the alert message from the individual CCRM, it establishes an open phone line with the center (MCC), certain algorithms and "hand-shake" procedures ensuring that a safe and correctly identified phone link is established with MCC. The transmission to the MCC contains the personal code of the patient and its receipt initiates access to his medical record, which is thus ready for comparison with the real-time data conveyed via the phone link.

Advantageously, at the end of the establishment of the phone link and unique patient identification, the radio link between the HCU and the CCRM is converted to a "hand-free" phone communication link, providing for voice communication between these units. A microphone is installed in the CCRM, providing for voice pickup anywhere on the premises where the patient is located, and for the transmission of the voice of the patient via his CCRM via radio link to the HCU, and from there via the phone link to the control center (MCC). The patient receives the required information and instructions via a loudspeaker located in the HCU and/or in the CCRM.

A critical item of information is the real-time ECG of the patient, and for this purpose the CCRM is equipped with two electrodes, and an ECG module. The ECG is obtained by the patient according to instructions from MCC and the data are transmitted to MCC via CCRM and HCU. The radio link between the CCRM and the HCU makes possible the taking of the ECG by the patient himself, wherever he is located in his dwelling during a heart attack or other cardiac symptoms and in the reliable transmission to the MCC, where the ECG is evaluated by medical personnel, based on the comparison with his previous medical records and ECGs.

Such evaluation makes possible to instruct the patient to take immediate steps required (such as an autoinjection of a drug provided for emergency use), and if required, a special mobile intensive care rescue unit is dispatched to transfer the patient to a medical care center.

The unique integrated system, comprising a radio link between the personal unit of the patient and a home control unit, which later is linked via a reliable phone link with a central control (MCC), makes possible to measure immediately critical physical parameters (pulse, flutter, arhythmia etc.) and a real-time ECG. Such immediate measurements make possible an immediate diagnosis at the central control unit (MCC), and initiation of the required rescue of the patient. The CCRM can also, via acoustic coupling to the phone connected to MCC, transmit at real time his ECG and other physical parameters to MCC.

### DESCRIPTION OF THE PREFERRED EMBODIMENT:

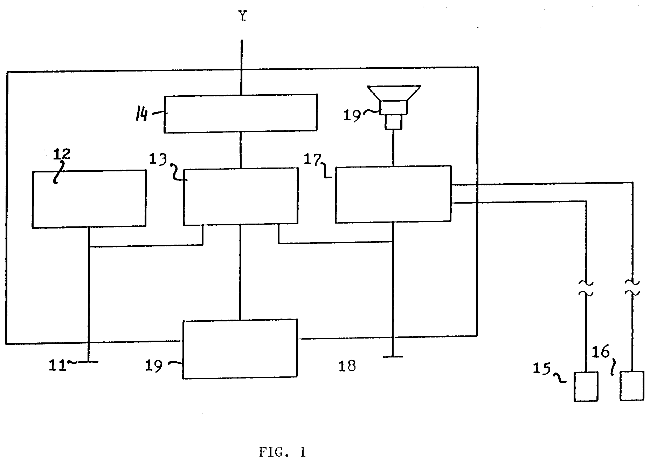

The invention is described by way of illustration only, with reference to enclosed Figure 1, which is a block diagram of a system of the invention.

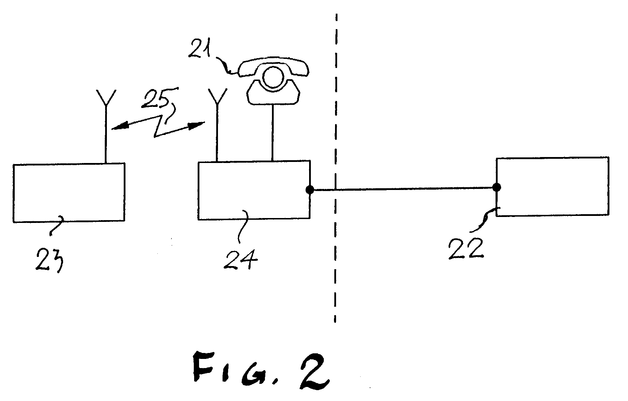

As shown in the Figure the CCRM, which is a small portable unit comprises in combination, a panic push button 11, connected via code generator 12 and logic unit 13 to transmitter 14. There are provided ECG electrodes 15 and 16, connected with ECG module 17, which is connected via logic unit 13 to the transmitter 14. There is provided a button 18, which serves to actuate the ECG measurement after attachment of the electrodes as required. The CCRM also includes a microphone 19, which is also connected via logic 13 to transmitter 14. An acoustic coupling 20 can be used for connection to the phone line;
As illustrated in Fig. 2, which is a block diagram of a system of the invention, this comprises a CCRM unit 23 as shown in Fig. 1, which is linked via a radio link 25 to HCU 24 and to phone 21, which provides a reliable link with central control MCC 22. The CCRM (Figure 1) is generally battery powered, and comprises means for monitoring whether the unit is in an operable condition. This is checked periodically by MCC so as to ascertain that this CCRM with function properly in an emergency.

The novel system provides all the required means for the acquisition of the important data during a caridac emergency, transmission to central control (MCC) and for providing both immediate temporary treatment and ambulance rescue of the afflicated person.

It is clear that the above description is by way of illustration only and that changes in the nature and arrangement of components and subunits can be resorted to without departing from the scope and spirit of the invention.

## Claims

1. An integrated medical events emergency rescue system, comprising in combination :
a medical control center (MCC) and means to establish contact by a patient from his premises with such MCC;
a portable rescue monitor unit (CCRM) comprising means for transmitting to MCC unique identifying data about the patient according to his personal code; means for measuring at least one critical parameter indicative of changes, of a change in the state of health of the said patient and also for vocal transmission to MCC;
a home control unit (HCU) connected via suitable communication means with said CCRM, said HCU being adapted to receive data from the CCRM and for transmitting same via phone link to MCC, there existing a link from MCC to HCU via CCRM enabling MCC to provide instructions via vocal means to the patient according to the results of an evaluation carried out at MCC on the basis of the transmitted information and means for initiating any required steps to provide aid to the patient in need thereof.

2. A system according to claim 1, where the CCRM is provided with an emergency push button, actuation of which establishes connection with central control, initiating the events required to provide aid to the patient.

3. An integrated cardiac emergency rescue system comprising in combination :
means for indicating to a control center (MCC) the occurrence of a change in cardiac symptoms :
a portable rescue monitor unit (CCRM) comprising means for transmitting to control (MCC) the personal code of the patient, electrode means attachable to the body of the patient, an ECG monitor and means for initiating the ECG measurement, means for transmitting the ECG data and/or other physical data from the CCRM unit to a home control unit (HCU) via a radio link; said HCU being connected with a phone, said HCU and phone being adapted to establish with a central control unit (MCC), a reliable phone link for transmitting the ECG and/or other data, and for receiving and transmitting to the CCRM any required information or instructions, means for diagnosing the status of the patient, means for initiating immediate emergency treatment by the patient or attending persons, and means for dispatching, if required, of a mobile intensive care unit to transfer the patient to a hospital.

4. A system according to any of claims 1 to 3, where the CCRM is integrated with the emergency push button.

5. A system according to any of claims 1 to 4, where the CCRM is battery-powered.

6. A system according to any of claims 1 to 5, where the monitor and control center (MCC) comprises means for comparing stored data to the patient with the real time data received via the HCU.

7. A system according to any of claims 1 to 6, where an acoustic link to the phone is provided.
